# FASCICULE DE BREVET EUROPEEN

(11) **EP 0 897 294 B1**
(45) Date de publication et mention de la délivrance du brevet: **28.11.2001**
(21) Numéro de dépôt: 97921913.6
(22) Date de dépôt: 30.04.1997
(51) Int. Cl.: A61F 2/16

(54) **IMPLANT INTRAOCULAIRE MONOBLOC SOUPLE**
EINTEILIGES VERFORMBARES INTRAOKULARLINSENIMPLANTAT
FLEXIBLE INTRAOCULAR IMPLANT FORMED IN ONE PIECE

(30) Priorité: 03.05.1996 FR 9605585
(43) Date de publication de la demande: 24.02.1999
(73) Titulaire: CORNEAL LABORATOIRES, 75012 Paris (FR)
(72) Inventeur: SOURDILLE, Philippe, F-44000 Nantes (FR); ORTUNO, Angel, F-74330 Choisy (FR)
(74) Mandataire: Dronne, Guy
(86) Numéro de dépôt international: FR9700772
(87) Numéro de publication internationale: WO9741805

(56) Documents cités:
- EP-A- 0 579 528
- EP-A- 0 766 952
- WO-A-94/28825
- FR-A- 2 701 390
- GB-A- 2 144 041
- GB-A- 2 180 160
- US-A- 4 787 904
- US-A- 5 133 749

## Description

La présente invention a pour objet un implant intraoculaire monobloc souple et notamment mais non exclusivement un implant de ce type destiné à être mis en place dans le sac capsulaire après ablation du cristallin.

Les implants intraoculaires peuvent se répartir en deux grandes catégories selon le matériau avec lequel ils sont réalisés. On distingue les implants intraoculaires dits souples et les implants intraoculaires dits rigides. Les premiers sont réalisés avec des matériaux du type gel de silicone ou pHEMA, les seconds étant réalisés en général à partir du PMMA.

Les implants rigides en PMMA sont développés et fabriqués depuis de nombreuses années et leur forme est parfaitement définie. En particulier, c'est le cas pour la forme des anses haptiques, ces anses ayant pour fonction de prendre appui sur la périphérie du sac capsulaire ou sur la paroi interne de l'oeil pour maintenir de façon élastique la partie optique de l'implant en regard de la pupille du patient qui a été implanté.

La tendance en matière de chirurgie oculaire et, plus précisément, de mise en place dans l'oeil d'implants intraoculaires est de réduire de façon très sensible la dimension de l'incision qu'il faut pratiquer dans la cornée pour mettre en place l'implant intraoculaire. Il faut ajouter que, l'implant est le plus souvent mis en place dans le sac capsulaire après l'ablation du cristallin, les techniques d'ablation du cristallin, notamment la phaco-émulsification, peuvent être mises en oeuvre en ne pratiquant qu'une incision de dimension réduite typiquement de l'ordre de 3 à 4 mm. Il était donc intéressant de disposer d'implants intraoculaires susceptibles d'être mis en place dans l'oeil à travers une telle incision. Cependant, on comprend que la dimension de la partie optique de l'implant, c'est-à-dire son diamètre, doit être suffisante pour que cette partie optique joue son rôle de correction, même lorsque la pupille est dilatée au maximum et malgré un éventuel décentrement de la partie optique. Il est donc nécessaire que la partie optique présente un diamètre suffisant typiquement de l'ordre de 5 à 6 mm.

Compte tenu de toutes ces contraintes, on comprend donc que seule l'utilisation d'un matériau souple permettant le pliage de la partie optique, permet de satisfaire à la double condition de passer par une incision dans la cornée de dimension réduite et de garantir un diamètre suffisant de la partie optique pour permettre la correction optique, quelle que soit la situation.

En conséquence, un grand nombre d'implants intraoculaires dits souples ont été développés pour satisfaire à ces deux conditions. Cependant, si les matériaux dits souples apportent une solution intéressante au problème de la réalisation de la partie optique, il n'en va pas de même pour ce qui concerne la réalisation de la partie haptique. En effet, la grande flexibilité de ces matériaux fait que la transposition directe de l'haptique des implants en matériau dur ne peut donner satisfaction dans le cas des matériaux souples.

Compte tenu de cette situation, deux grandes types de solution ont été proposés: d'une part, on a proposé de réaliser des implants dans lesquels la partie optique est souple et la partie haptique est rigide typiquement réalisé en PMMA. Ces techniques sont notamment décrites dans la demande de brevet WO-A-96/11792. Cette solution permet, d'une part de bénéficier d'une optique souple pliable et, d'autre part, d'utiliser des anses haptiques en PMMA dont la définition géométrique est parfaitement maîtrisée.

On a également proposé de réaliser la partie haptique pour les implants souples, non pas par l'intermédiaire de deux anses en C ou en J disposés diamétralement par rapport à l'optique, mais en prévoyant deux parties haptiques beaucoup plus massives se terminant par des rebords en arc-de-cercle de longueur suffisante pour assurer un contact suffisant entre la périphérie du sac capsulaire et la partie haptique. De tels implants souples sont notamment décrits dans la demande de brevet européen EP-A-0 579 528.

Le document **D1=: GB-A-2180160** décrit une lentille intraoculaire à centrage automatique. L'implant intraoculaire selon le document **D1** comprend une partie optique de forme sensiblement circulaire et deux anses haptiques courbes présentant chacune une première extrémité de raccordement et une deuxième extrémité libre; la partie optique et les anses haptiques sont réalisées avec un même matériau et la largeur dans le plan optique de chaque anse va en diminuant de son extrémité de raccordement à son extrémité libre. Dans l'état implanté dans l'oeil, le matériau de l'implant est souple.

On a néanmoins déjà proposé de réaliser des implants monoblocs souples avec une partie haptique constituée par deux anses courtes de type relativement classique. Cependant, ces implants sont peu satisfaisants dans la mesure où, en raison de la très grande flexibilité de ce matériau qui présente un module d'élasticité typiquement inférieur à 0,2 MPa, l'anse haptique, sous l'effet des contraintes résultant de sa mise en place dans le sac capsulaire, se plie localement dans la région du raccordement de l'anse à la périphérie de la partie optique. Il en résulte que la zone de contact effective entre les anses haptiques et la périphérie du sac capsulaire est réduite, ce qui assure imparfaitement le maintien en place de la partie optique et ce qui risque surtout d'entraîner une déformation du sac capsulaire avec les conséquences dommageables que cela entraîne ou même une perforation de ce sac capsulaire sous l'effet de la concentration des contraintes de pression. c'est ce qui est représenté sur la figure 6. la partie optique est référencée 2, les anses haptiques 4 et la périphérie du sac capsulaire 6.

Un objet de la présente invention est de fournir un implant intraoculaire monobloc souple présentant une partie haptique constituée par des anses mais qui comporte une partie haptique ayant des propriétés de maintien et de flexibilité améliorées pour obtenir des résultats sensiblement équivalents à ceux que l'on obtient avec des anses haptiques en PMMA.

Pour atteindre ce but, l'implant intraoculaire qui comprend une partie optique de forme sensiblement circulaire et deux anses haptiques courbes présentant chacune une première extrémité de raccordement à la périphérie de la partie optique et une extrémité libre destinée à venir en appui sur la paroi interne de l'oeil se caractérise en ce que la partie optique et les anses haptiques sont réalisés avec un même matériau souple dont le module d'élasticité est compris entre 0,25 MPa et 1 MPa, et en ce que la largeur dans le plan optique de chaque anse va en diminuant de son extrémité de raccordement à son extrémité libre de telle manière que la variation du rapport entre la variation du moment de flexion appliqué à l'anse et la variation du moment d'inertie soit sensiblement constante entre deux points distincts de l'anse sur toute la longueur de l'anse.

Grâce à cette définition spécifique de la géométrie de l'anse, on obtient une flexion répartie de l'anse qui lui permet ainsi de s'adapter par flexion aux différents diamètres internes du sac capsulaire en évitant une flexion localisée de l'anse au niveau de son raccordement à la partie optique et en réalisant ainsi une longueur de contact importante entre l'anse et la paroi interne du sac capsulaire ou de l'oeil selon les modes d'implantation.

Selon un mode préféré de réalisation, chaque anse haptique comprend en outre un bras complémentaire séparé de l'anse proprement dite et dont une première extrémité est raccordée à la périphérie de la partie optique à proximité de l'extrémité de raccordement de l'anse et dont l'autre extrémité est raccordée à l'anse à proximité de son extrémité libre, la section dudit bras additionnel étant inférieure à celle de l'anse, ledit bras étant disposé du côté concave de l'anse.

Grâce à la présence d'un bras complémentaire associé à chaque anse haptique proprement dite, non seulement on conserve les propriétés de flexion des anses conformes à la définition donnée précédemment mais, en outre, on augmente le moment d'inertie de chaque anse haptique par rapport aux risques de torsion de l'anse par rapport à sa fibre neutre. Cela permet ainsi d'éviter un mouvement de rotation relatif de la partie optique par rapport aux deux anses, ce mouvement de rotation risquant de produire un déplacement de la partie optique de telle manière que le plan de la partie optique ne soit plus perpendiculaire à l'axe optique de l'oeil.

D'autres caractéristiques et avantages de l'invention apparaîtront mieux à la lecture de la description qui suit de plusieurs modes de réalisation de l'invention donnés à titre d'exemples non limitatifs. La description se réfère aux figures annexées sur lesquelles :
- la figure 1a est une vue de face d'un premier mode de réalisation de l'implant intraoculaire souple;
- la figure 1b est une vue de côté de l'implant intraoculaire de la figure 1a;
- la figure 2a est une vue de face d'un deuxième mode de réalisation de l'implant intraoculaire souple;
- la figure 2b est une vue de côté de l'implant intraoculaire de la figure 2a;
- la figure 3 est une vue de l'implant intraoculaire de la figure 1a mis en place dans le sac capsulaire;
- les figures 4a et 4b montrent un implant intraoculaire du type représenté sur la figure 2a mis en place dans des sacs capsulaires de diamètre différent;
- les figures 5a à 5c sont des courbes montrant la force de compression en fonction du diamètre final de l'implant après sa mise en place dans le sac capsulaire; et
- la figure 6 déjà décrite montre un implant souple de l'art antérieur mis en place dans le sac capsulaire.

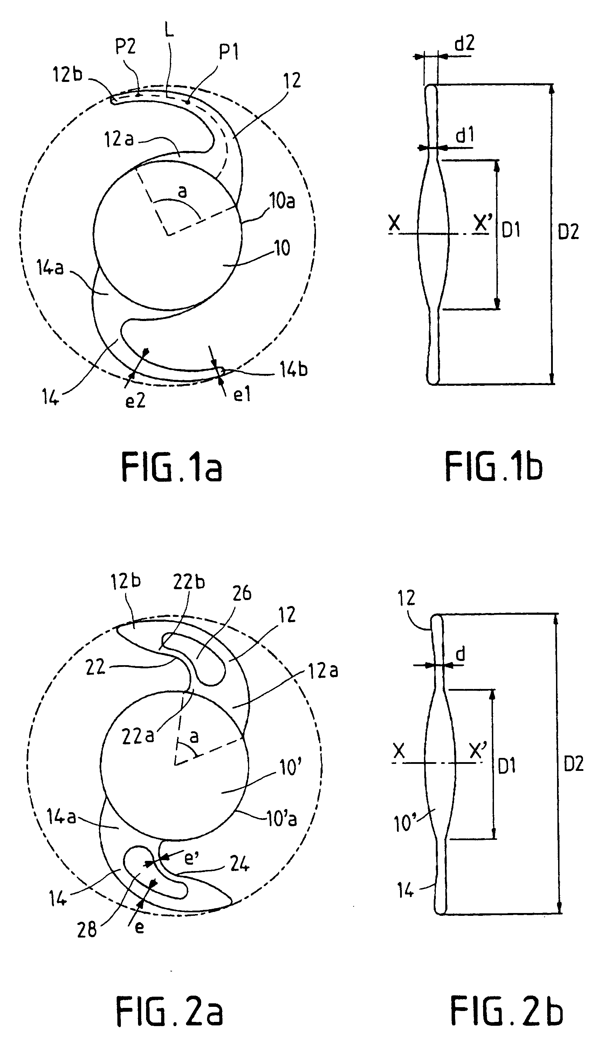

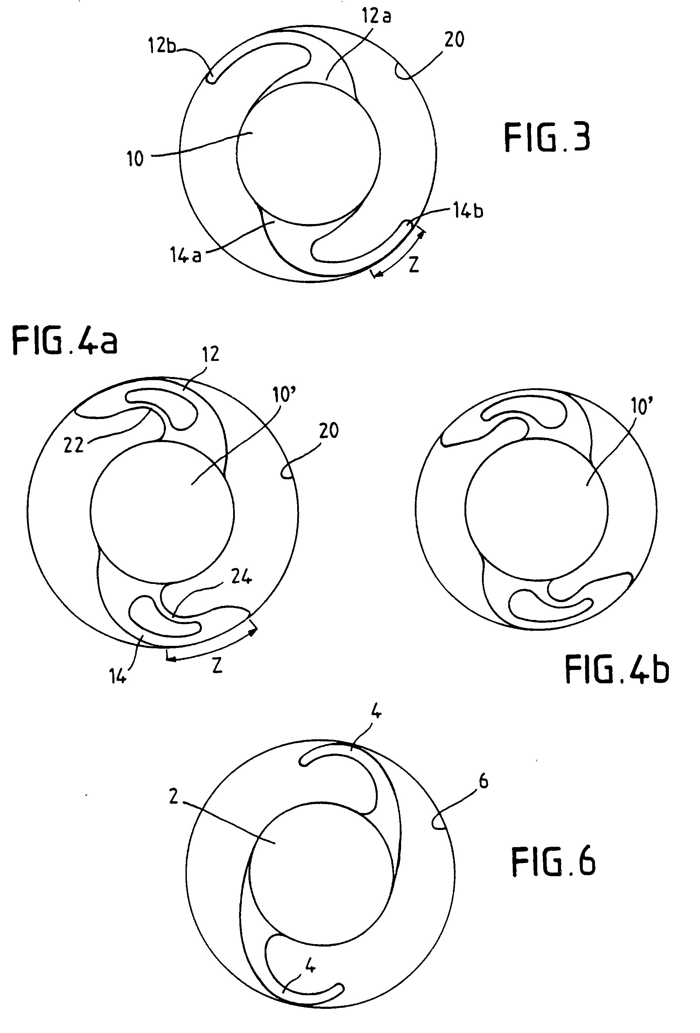

En se référant tout d'abord aux figures 1a et 1b ainsi qu'à la figure 3, on va décrire un premier mode de réalisation de l'implant intraoculaire monobloc souple. Comme le montre la figure 1a, l'implant intraoculaire est constitué par une partie optique 10 de forme sensiblement circulaire et limitée par un dioptre antérieur et un dioptre postérieur et par une partie haptique constituée par deux anses 12 et 14, ces deux anses étant identiques ou sensiblement identiques et raccordées à la périphérie 10a de la partie optique en deux points sensiblement diamétralement opposés. Ces anses en C présentent une courbure qui varie de façon régulière sans présenter de points singuliers.

Selon une caractéristique essentielle de l'invention, l'implant est monobloc, c'est-à-dire que la partie optique 10 et les anses haptiques 12 et 14 ne constituent qu'une seule pièce, cette pièce étant un pHEMA, par exemple de l'hydrogel. Le module d'élasticité de la composition de pHEMA utilisé est compris entre 0,25MPa et 1MPa. De préférence, selon le mode particulier de réalisation décrit, le module d'élasticité est égal à 0,6 MPa. On voit donc que, pour réaliser l'implant monobloc souple, on utilise un type de pHEMA présentant un module d'élasticité supérieur au pHEMA classiquement utilisé. Cependant, en limitant le module d'élasticité à 1MPa, on conserve la possibilité de plier l'optique aisément et de maintenir l'optique pliée avec une force de maintien compatible avec l'intervention chirurgicale.

En outre, comme on le voit, chaque anse 12 comporte une zone, une extrémité de raccordement 12a qui présente une largeur importante par rapport à la partie courante de l'anse et qui est raccordée donc sur un angle au centre a de la partie optique relativement importante, par exemple de l'ordre de 80 degrés et plus généralement compris entre 60 et 90 degrés, et une deuxième extrémité libre 12b destinée à venir en appui sur la paroi interne du sac capsulaire ou de l'oeil selon le mode d'implantation retenu.

Selon une caractéristique essentielle de l'invention, si l'on considère la fibre neutre L de l'anse et si l'on considère deux points P1 et P2 de cette fibre neutre, la géométrie de la partie courante de l'anse est réalisée de telle manière que le rapport de la variation du moment de flexion présent aux points P1 et P2 dû à l'effort appliqué sur l'anse lors de sa mise en place dans l'oeil, et de la variation du moment d'inertie en ces deux points P2 et P1, soit sensiblement constante.

Pour ce faire, et si l'on considère une force exercée sur le bout de l'anse, alors le moment de flexion va croître au fur et à mesure que l'on se rapproche de l'optique de façon sensiblement linéaire. Il s'agit donc de faire varier le moment d'inertie de la même façon. Le moment d'inertie va s'exprimer de la façon suivante : I₂ = I₀ + 1 x ΔI.

I₀ est le moment d'inertie en bout d'anse, 1 étant la distance (en mm) qui sépare le bout de l'anse au point considéré, cette distance étant prise sur ladite fibre neutre.

ΔI est le coefficient de variation du moment d'inertie. Cette variation de moment d'inertie est exprimée en mm⁴. De préférence, ΔI est compris entre 5.10⁻⁴ mm³ et 15.10⁻⁴ mm³.

Grâce à cette disposition, on obtient ainsi une courbure par flexion régulière de l'anse lorsque l'implant est mis en place à l'intérieur du sac capsulaire. Par exemple, sur la figure 1a-1b, le diamètre D1 de la partie optique est de 6 mm alors que le diamètre D2 externe de la partie haptique au repos est égal à 12 mm.

Sur la figure 3, on a représenté la déformation de l'anse lorsque l'implant est mis en place dans un sac capsulaire 20 de diamètre interne 11 mm. On voit que la flexion de chaque anse, au lieu de se concentrer dans les zones de raccordement 12a à 14a, se développe sur l'ensemble de la longueur de l'anse 12-14, ce qui permet d'avoir une zone de contact importante Z entre la partie de l'anse proche de son extrémité libre 12b-14b avec la paroi interne du sac capsulaire.

On comprend que cette longueur relativement importante de contact évite une déformation localisée du sac capsulaire, du fait que la pression ponctuelle appliquée se répartit sur toute la zone Z et non en un nombre de points limité, comme dans le cas des implants de l'état de la technique. En outre, cette disposition permet d'éviter les risques de perforation de sac capsulaire du fait d'une pression localisée importante. Enfin, du fait que cette zone Z présente une longueur relativement importante, on a un bon appui de chaque anse sur la paroi interne du sac capsulaire et donc un bon maintien en place de la partie optique.

Pour obtenir cette constance ou sensiblement constance du rapport de la variation du moment de flexion appliqué à l'anse sur la variation du moment d'inertie, de préférence, l'anse a dans le plan optique une largeur e qui va en diminuant de son extrémité de raccordement 14a vers son extrémité libre 14b. Sur la figure 1a, on a représenté une largeur e1 proche de la zone de raccordement égal à 0,55 mm et une largeur e2 à son extrémité 14b qui vaut 0,35 mm. Comme le montre la figure 1b, en revanche, si l'on considère l'épaisseur d de chaque anse, c'est-à-dire sa dimension selon la direction de l'axe optique XX', cette épaisseur d va en augmentant de la périphérie de la partie optique vers l'extrémité libre. Typiquement, dans la zone d1, cette épaisseur est de 0,35 mm alors qu'à l'extrémité libre 12b, cette épaisseur d2 vaut 0,45 mm. Pour ce faire le moment d'inertie varie en fonction de la distance qui sépare le bout de l'anse au point considéré sur la fibre neutre avec un coefficient compris entre 5.10⁻⁴ et 15.10⁻⁴. Le moment est exprimé en mm⁴ et la distance en mm.

En se référant maintenant aux figures 2a, 2b et 4a, 4b, on va décrire un deuxième mode de réalisation de l'implant intraoculaire monobloc souple selon l'invention. Dans ce mode de réalisation, chaque anse haptique 12, 14 et qui a, dans la partie courante, les mêmes dimensions que les anses des modes de réalisation de la figure 1a, est complétée par un bras additionnel respectivement référencé 22, 24. Chaque bras 22, 24 comporte également une extrémité de raccordement 22a à la périphérie de la partie optique et une extrémité 22b de raccordement à l'extrémité libre 12b de l'anse associée. Le bras 22 ou 24 a une largeur sensiblement constante e' sauf bien sûr dans sa partie d'extrémité de raccordement 22b où cette épaisseur est plus importante pour assurer la continuité avec l'anse 12, la largeur e' est typiquement égale à 0,25 mm. Ainsi, l'ensemble constitué par l'anse 12 et le bras 22 définit entre ces deux éléments un évidement respectivement référencé 26 et 28.

Ce mode de réalisation perfectionné présente tous les avantages du mode de réalisation de la figure 1a, en ce qui concerne les qualités de flexion des anses proprement dites 12 et 14 mais, en outre, le couplage mécanique des bras 22 et 24 avec les anses 12 et 14 permet d'éviter les risques de rotation de la partie optique 10, en raison des risques de torsion des anses 12 et 14, du fait de leur constitution en un matériau relativement souple. En effet, les bras additionnels 22 et 24 étant directement reliés à une autre zone de la périphérie de la partie optique et à l'extrémité libre de chaque anse, ce mouvement de torsion est évité ou, du moins, les risques en sont considérablement diminués.

Les figures 4a et 4b montrent la mise en place de l'implant intraoculaire 10' de la figure 2a dans un sac capsulaire 20 dont le diamètre interne est respectivement égal à 11 mm pour la figure 4a et 10 mm pour la figure 4b. Ces figures montrent en particulier la déformation par flexion des anses 12 et 14 et des bras 22 et 24. On constate que l'on a encore une zone Z relativement importante de contact entre les anses haptiques et la périphérie 20 du sac capsulaire, ce qui présente bien sûr les mêmes avantages que ceux qui ont été décrits en liaison avec la figure 3.

Les figures Sa à 5c montrent les essais effectués respectivement sur un implant souple monobloc de l'art antérieur (5a), et sur les implants de la figure 1a (5b) et de la figure 2a (5c). Sur chaque diagramme, on représente la force de compression F en fonction du diamètre externe D de la partie haptique, le diamètre D au repos étant égal à 12 mm.

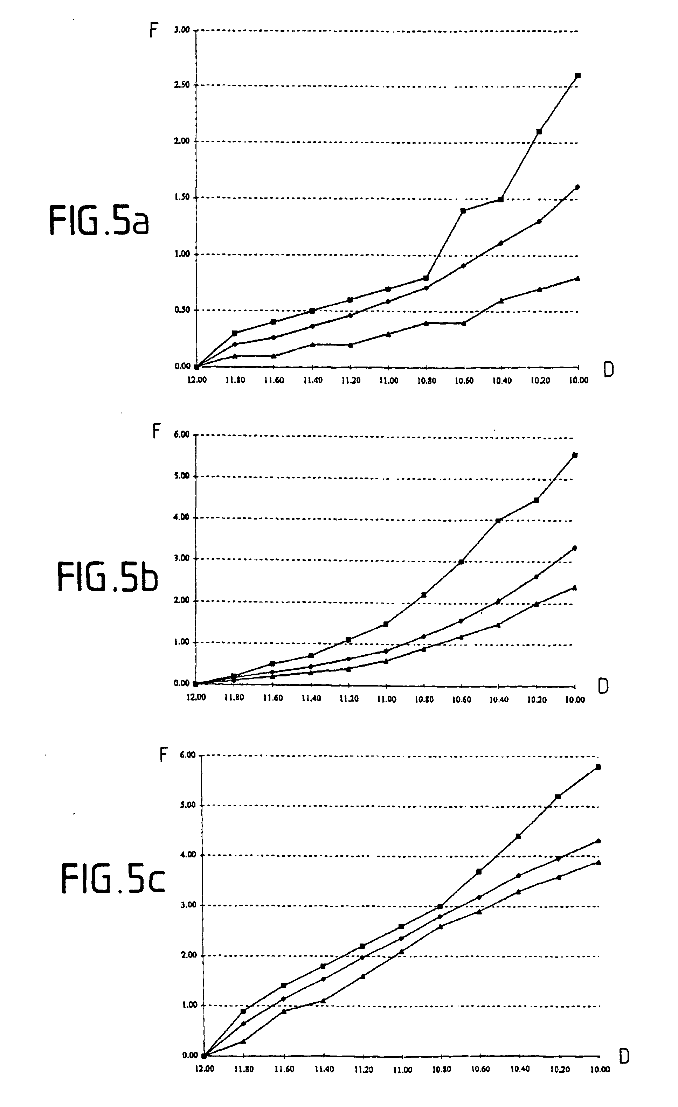

Dans le cas de la figure 5a, on voit que la force de compression est très réduite, alors que, dans le cas des figures 5b et 5c, cette force de compression est bien plus élevée pour un même diamètre, ce qui assure un bien meilleur maintien en place dans l'oeil de la partie optique.

## Revendications

1. Implant intraoculaire comprenant une partie optique (10) de forme sensiblement circulaire et deux anses haptiques courbes (12,14) présentant chacune une première extrémité de raccordement à la périphérie (10a) de la partie optique et une deuxième extrémité libre destinée à venir en appui sur la paroi interne de l'oeil, la partie optique et les anses haptiques étant réalisées avec un même matériau souple, **caractérisé en ce que** le module d'élasticité dudit matériau souple est compris entre 0,25 MPa et 1 MPa et **en ce que** la largeur de chaque anse va en diminuant de son extrémité de raccordement (12a,14a) à son extrémité libre (12b,14b) de telle manière que le rapport entre la variation du moment de flexion et la variation du moment d'inertie en deux points distincts (P1, P2) de l'anse soit sensiblement constant.

2. Implant intraoculaire selon la revendication 1, **caractérisé en ce que** la variation du moment d'inertie rapportée à la distance qui sépare la deuxième extrémité (12b, 14b) de l'anse du point considéré sur la fibre neutre de l'anse est comprise entre 5.10⁻⁴ mm³ et 15.10⁻⁴ mm³, le moment d'inertie étant exprimé en mm⁴ et la distance en mm.

3. Implant intraoculaire selon l'une quelconque des revendications 1 et 2, **caractérisé en ce que** chaque anse haptique comprend en outre un bras complémentaire (22,24) separé de l'anse proprement dite et dont une première extrémité est raccordée à la périphérie de la partie optique à proximité de l'extrémité de raccordement de l'anse et dont une deuxième extrémité est raccordée à l'anse à proximité de son extrémité libre, la section droite dudit bras complémentaire étant inférieure à celle de l'anse, ledit bras étant disposé du côté concave de l'anse.

4. Implant intraoculaire selon la revendication 3, **caractérisé en ce que** l'épaisseur (d) de chaque anse selon la direction de l'axe optique va en augmentant de l'extrémité de raccordement vers l'extrémité libre.

5. Implant intraoculaire selon l'une quelconque des revendications 1 à 4, **caractérisé en ce que** l'extrémité de raccordement de chaque anse (12a, 14a) s'étend sur la périphérie (10a) de la partie optique (10) sur une longueur correspondant à un angle au centre (a) compris entre 60 et 90 degrés.

## Claims

1. Intraocular implant comprising a substantially circular optical portion (10) and two curved haptic loops (12, 14) each with a first connecting end at the periphery (10a) of the optical portion and a free second end intended to come to rest on the internal wall of the eye, the optical portion and the haptic loops being made of the same flexible material, **characterized in that** the modulus of elasticity of said flexible material is included between 0.25 MPa and 1 MPa, and **in that** the width of each loop decreases from its connecting end (12a, 14a) to its free end (12b, 14b) such that the ratio between the variation of the bending moment and the inertia moment variation is substantially constant between two separate points (P1, P2) of the loop.

2. Intraocular implant according to Claim 1, **characterized in that** the inertia moment variation divided by the distance which separates the second end (12b, 14b) of the loop from the point in question on the neutral axis of the loop is included between 5.10⁻⁴ mm³ and 15.10⁻⁴ mm³, the inertia moment being expressed in mm⁴ and the distance in mm.

3. Intraocular implant according to Claim 1 or 2, **characterized in that** each haptic loop further comprises a complementary arm (22, 24) separated from the loop proper and of which a first end is connected to the periphery of the optical portion near the connecting end of the loop and of which a second end is connected to the loop near its free end, the cross section of said complementary arm being smaller than that of the loop, said arm being disposed on the concave side of the loop.

4. Intraocular implant according to Claim 3, **characterized in that** the thickness (d) of each loop in the direction of the optical axis increases from the connecting end towards the free end.

5. Intraocular implant according to any one of Claims 1 to 4, **characterized in that** the connecting end of each loop (12a, 14a) extends on the periphery (10a) of the optical portion (10) over a length corresponding to an angle at the centre (a) included between 60 and 90 degrees.

## Patentansprüche

1. Intraokularimplantat, das ein optisches Teil (10) mit im wesentlichen kreisförmiger Form und zwei gekrümmte haptische Bügel (12, 14) aufweist, die jeweils ein erstes Ende zur Verbindung am Rand (10a) des optischen Teils und ein freies zweites Ende aufweisen, das dazu bestimmt ist, gegen die Innenwand des Auges zu drücken, wobei das optische Teil und die haptischen Bügel mit demselben nachgiebigen Material hergestellt sind, **dadurch gekennzeichnet, dass** der Elastizitätsmodul des nachgiebigen Materials zwischen 0,25 MPa und 1 MPa beträgt, und dass die Breite jedes Bügels sich von seinem Verbindungsende (12a, 14a) zu seinem freien Ende (12b, 14b) derart verringert, dass das Verhältnis zwischen der Veränderung des Biegemoments und der Veränderung des Trägheitsmoments in zwei verschiedenen Punkten (P1, P2) des Bügels im wesentlichen konstant ist.

2. Intraokularimplantat nach Anspruch 1, **dadurch gekennzeichnet, dass** die Veränderung des Trägheitsmoments bezogen auf den Abstand, der das zweite Ende (12b, 14b) des Bügels vom betrachteten Punkt auf der neutralen Faser des Bügels trennt, zwischen 5 X 10⁻⁴ mm³ und 15 X 10⁻⁴ mm³ beträgt, wobei des Trägsheitsmoment in mm⁴ und der Abstand in mm ausgedrückt sind.

3. Intraokularimplantat nach einem der Ansprüche 1 und 2, **dadurch gekennzeichnet, dass** jeder haptische Bügel außerdem einen vom eigentlichen Bügel getrennten, komplementären Arm (22,24) aufweist, und dass ein erstes Ende von diesem mit dem Rand des optischen Teils in der Nähe des Verbindungsendes des Bügels verbunden ist, und dass ein zweites Ende mit dem Bügel in der Nähe seines freien Endes verbunden ist, wobei der Querschnitt des komplementären Arms kleiner ist als derjenige des Bügels, wobei der Arm an der konkaven Seite des Bügels angeordnet ist.

4. Intraokularimplantat nach Anspruch 3, **dadurch gekennzeichnet, dass** die Dicke (d) jedes Bügels in Richtung der optischen Achse sich vom Verbindungsende zum freien Ende hin vergrößert.

5. Intraokularimplantat nach einem der Ansprüche 1 bis 4, **dadurch gekennzeichnet, dass** das Verbindungsende jedes Bügels (12a, 14a) sich auf dem Rand (10a) des optischen Teils (10) auf einer Länge erstreckt, die einem Winkel im Zentrum (a) zwischen 60 und 90 Grad entspricht.
